# EUROPÄISCHE PATENTSCHRIFT

(11) **EP 2 355 855 B1**
(45) Veröffentlichungstag und Bekanntmachung des Hinweises auf die Patenterteilung: **17.09.2014**
(21) Anmeldenummer: 09810761.8
(22) Anmeldetag: 06.11.2009
(51) Int. Cl.: A61L 2/10, D06F 35/00, F21K 99/00, C02F 1/32, H01J 61/44, H05B 33/14, H01L 33/50, D06F 39/00

(54) **UV-DESINFEKTIONSVORRICHTUNG FÜR TEXTILIEN, INSBESONDERE WASCHKUGEL, DEREN VERWENDUNG ZUM DESINFIZIEREN VON TEXTILIEN.**
UV DISINFECTING DEVICE FOR NFECTING TEXTILES, PARTICULARLY LAUNDRY BALL, THE USE THEREOF FOR DISINFECTING TEXTILES
DISPOSITIF POUR DÉSINFECTION PAR UV DE TEXTILES, EN PARTICULIER BOULE DE LAVAGE , SON UTILISATION POUR LA DÉSINFECTION DE TEXTILES

(30) Priorität: 06.11.2008 DE 102008056497; 05.02.2009 DE 102009008031
(43) Veröffentlichungstag der Anmeldung: 17.08.2011
(73) Patentinhaber: Trossowski, Peter, 10715 Berlin (DE)
(72) Erfinder: Trossowski, Peter, 10715 Berlin (DE)
(86) Internationale Anmeldenummer: PCT/DE2009/001616
(87) Internationale Veröffentlichungsnummer: WO 2010/051808

(56) Entgegenhaltungen:
- WO-A1-00/79246
- WO-A1-98/32700
- WO-A1-2007/058478
- CH-A5- 625 284
- DE-A1- 3 123 899
- US-A1- 2008 035 581

## Beschreibung

Die vorliegende Erfindung betrifft ein System zur Desinfektion (Bedeutung: "Totes oder lebendes Material in einen Zustand versetzen, so dass es nicht mehr infizieren kann") von Wäsche und/oder wäscheähnlichen Gebrauchsgegenständen durch den Einsatz von UV-Licht- bzw. Strahlungsquellen, insbesondere durch UV-LED's. Einsatzgebiete der Erfindung sind (unter anderem) insbesondere Privathaushalte, gewerbliche Wäschereien sowie Kranken- und Pflegeeinrichtungen, bspw. Krankenhäuser und Pflegeheime.

UV-Sterilisationsvorrichtungen geeignet zum Einsatz in Waschmaschinen sind aus den Druckschriften US2008035581 und WO9832700.

Momentan wird die Desinfektion durch einen Wäschezusatz aus verschiedenen Chemikalien erreicht. Dieser Zusatz wird in der Regel zum letzten Waschgang (dem des Weichspülens), zusammen mit dem Weichspüler, der Wäsche hinzugefügt. Hier entfaltet er seine desinfizierende Wirkung.

Diese Zusatzstoffe werden in der Waschmaschine mit dem vorhandenen Waschwasser vermischt. Nachteilig an diesem Vorgehen ist u.a., dass hierdurch eine hohe Konzentration des Desinfektionsmittels benötigt wird, welche wiederum mit dem Schmutzwasser in die Kanalisation gelangt und eine unnötige Belastung des Abwassers darstellt.

Aufgabe der Erfindung ist daher die Bereitstellung eines Desinfektionssystems für Textilien und textilienähnlichen Gebrauchsgegenständen, das diese Nachteile nicht aufweist. Diese Aufgabe wird gemäß den Ansprüchen gelöst. Die Erfindung betrifft demnach eine Vorrichtung zum Desinfizieren von Textilien gemäß Anspruch 1, ihre Verwendung gemäß Anspruch 14. Die weiteren Ansprüche betreffen bevorzugte Ausführungsformen der Erfindung.

Überraschenderweise hat sich eine bewegliche Vorrichtung nach Anspruch 1 als besonders vorteilhaft zum Desinfizieren von Textilien erwiesen.

Die UV-Licht emittierenden Strahlungsquellen (2) gemäß der Erfindung sind dabei vorzugsweise als UV-Licht emittierende Leuchtkörper, vorzugsweise Quecksilberdampflampen bzw. -röhren oder Schwarzlichtlampen (Glühlampen mit Filter oder Quecksilberdampf-Niederdrucklampen mit Filter und Leuchtstoff für UV-A) oder besonders bevorzugt als UV-Licht emittierende Dioden, nachfolgend auch UV-LEDs genannt, gebildet.

Die Erfindung basiert also auf einem Prozess, der sich beim Leuchten von UV-Licht- bzw. Strahlungsquellen, insbesondere UV-LEDs, ergibt. Die hierbei entstehenden Lichtwellen erzeugen eine desinfizierende Wirkung. Totes oder lebendes Material, welches sich in den zu waschenden Objekten befindet, wird insbesondere durch irreversible Schädigung seines Erbgutes in einen Zustand versetzt, in dem es nicht mehr infektiös wirken kann.

Die elektrisch leitende Verbindungsmittel (7) sind vorzugsweise als Drähte oder besonders bevorzugt als Kabel gebildet.

Nach einem Aspekt der Erfindung sind also UV-LEDs (2), Verbindungskabel und ein Akkumulator (3) in einem Gehäuses bzw. mittels eines Gehäuses gelagert und derart angeordent und miteinander verbunden, dass die Vorrichtung mittels der UV-LEDs (2) UV-Licht in die Umgebung der Vorrichtung emittieren kann. Die Vorrichtung ist also derart ausgestaltet, dass sie räumlich frei bewegt werden kann und während ihrer Bewegungen UV-Licht in ihre äußere Umgebung emittieren kann, das dann in der Nähe befindliche Textilien und ggf. an die Vorrichtung angrenzende Waschlösung zumindest teilweise durchdringt.

Die UV-LEDs (2), die Verbindungskabel und der Akkumulator (3) werden dabei vorteilhafterweise durch das Gehäuse (1) wechselseitig räumlich zueinander fixiert, so dass die Abstände dieser Bestandteile auch bei der vorgesehenen Bewegung der Vorrichtung konstant zueinander gehalten sind.

Das Gehäuse ist vorzugsweise aus im Wesentlichen nicht elektrisch leitendem Material, insbesondere aus PVC, Haushaltskunststoff, Teflon, Epoxydharz, Kevlar oder einer Kombination dergleichen, gebildet. Besonders geeignet sind dabei UV-Licht durchlässige Kunststoffe, insbesondere wenn das Gehäuse aus PET und/oder Plexiglas gebildet ist.

Besonders geeignet ist eine Vorrichtung, die mehr als zwei UV-Licht emittierenden Strahlungsquellen, vorzugsweise UV-LEDs, aufweist, insbesondere zwischen 4 und 36, vorzugsweise zwischen 6 und 24, insbesondere bevorzugt zwischen 12 und 20 UV-Licht emittierenden Strahlungsquellen, vorzugsweise UV-LEDs..

In einer besonders vorteilhaften Ausführungsform der Vorrichtung ist das Gehäuse (1) als dreidimensionaler Körper gebildet, der Aussparungen zur Aufnahme der UV-Licht emittierenden Strahlungsquellen (2), der elektrisch leitenden Verbindungsmittel (7) und des Akkumulators (3) aufweist.

In einer weitem besonders vorteilhaften Ausführungsform der Vorrichtung befinden sich In oder Auf dem Gehäuse (1) das als dreidimensionaler Körper gebildet ist, ein oder mehrere Prismen derart angeordnet das dass UV-Licht gestreut wird.

In einer anderen vorteilhaften Ausführungsform der Vorrichtung ist das Gehäuse (1) aus mehreren, insbesondere zwei, Teilen zusammengesetzt, vorzugsweise aus einer Oberschale und einer Unterschale.

In einer weiteren vorteilhaften Ausführungsform der Vorrichtung sind die UV-Licht emittierenden Strahlungsquellen (2) und/oder ist der Akkumulator (3) im wesentlichen von dem Gehäuse (1) umgeben, wobei wenigstens ein Teil des Akkumulators (3) aus dem Gehäuse (1) herausragt oder nicht mit dem Gehäuse (1) in Berührung ist, so dass ein Anschluss des Akkumulators (3) an eine vorzugsweise extern gelagerte/aufgestellte Ladevorrichtung (4) ermöglicht ist. Bevorzugt sind die UV-Licht emittierenden Strahlungsquellen (2) derart von dem Gehäuse umgeben, dass kein direkter Kontakt der Bauteile der UV-Licht emittierenden Strahlungsquellen (2) mit der äußeren Umgebung der Vorrichtung stattfindet.
In einer anderen bevorzugten Ausführungsform der Vorrichtung ist das Gehäuse (1) als Kugel gebildet, wobei vorzugsweise das Gehäuse (1) im Bereich des für den Anschluss an eine (externe) Ladevorrichtung vorgesehenen Teils des Akkumulators (3) flach/abgeschrägt ausgebildet ist. Somit ist die die Vorrichtung als UV-Desinfektionswaschkugel ausgebildet, wobei vorteilhafterweise der Radius des kugelförmigen Gehäuses (1) dem üblichen Radius von gebräuchlichen Waschkugeln zum Dosieren von Waschmittel entspricht.

In einer weiteren bevorzugten Ausführungsform der Vorrichtung ist das Gehäuse (1) aus vorzugsweise durchsichtigem Kunststoff, insbesondere aus Polyethylenterephthalat (PET) oder Polymethylmethacrylat (Plexiglas), gebildet.

In wiederum einer anderen vorteilhaften Ausführungsform der Vorrichtung ist das von dem UV-Licht emittierenden Strahlungsquellen emittierbare Licht im wesentlichen UV-A-, UV-B-und/oder UV-C-Licht.

In wiederum einer weiteren bevorzugten Ausführungsform umfasst oder beinhaltet die Vorrichtung ferner eine Steuerelektronik zum Steuern und/oder Regeln der UV-Licht emittierenden Strahlungsquellen (2).

Die Vorrichtung beinhaltet vorzugsweise ferner eine zumindest teilweise elektrisch leitende Halterung für den Akkumulator (3), insbesondere ein übliches Akkuaufnahmegehäuse, wobei die Steuerelektronik besonders bevorzugt in der Halterung integriert ist.

In wiederum einer anderen besonders vorteilhaften Ausführungsform umfasst oder beinhaltet die Vorrichtung ferner eine Ladevorrichtung (4), die vorzugsweise eine Induktionsladevorrichtung ist.

In wiederum einer weiteren besonders vorteilhaften Ausführungsform umfasst oder beinhaltet die Vorrichtung ferner eine Ladevorrichtung zum Laden des Akkumulators (3) während des Einsatzes. Dies wird durch einen Rotor (Exzenterstück) erreicht, der auf einer Generatorwelle befestigt ist und bei Bewegung der Vorrichtung den Exzenter auf der Welle in Rotation versetzt. Hierdurch wird im Generator eine Ladespannung erzeugt, die den Akkumulator (3) auflädt.

In einer besonders einfach zu realisierenden Ausführungsform umfasst oder beinhaltet die Vorrichtung ferner einen Schalter zum Ein- und/oder Ausschalten der Vorrichtung, wobei der Schalter vorzugsweise im Bereich der Oberfläche des Gehäuses (1) angeordnet ist. Der Schalter ist insbesondere derart mit den UV-Licht emittierenden Strahlungsquellen (2) und dem Akkumulator (3) verbunden, dass über den Schalter ein Stromkreislauf über die UV-Licht emittierenden Strahlungsquellen (2) und den Akkumulator (3) herstellbar ist und/oder unterbrochen werden kann.
In einer weiteren besonders einfach zu realisierenden Ausführungsform umfasst oder beinhaltet die Vorrichtung ein System zum Ein- und/oder Ausschalten der Vorrichtung, wobei der Impuls für die Schaltung über ein Funk- und/oder Infrarotsystem erfolgt. Das Schaltsystem ist insbesondere derart mit der Steuerelektronik (5), den UV-Licht emittierenden Strahlungsquellen (2) und dem Akkumulator (3) verbunden, dass mittels des den Impulsgebers der Stromkreislauf über die UV-Licht emittierenden Strahlungsquellen (2) und den Akkumulator (3) herstellbar ist und/oder unterbrochen werden kann.

Ein weiterer Aspekt der Erfindung betrifft die Verwendung der erfindungsgemäßen Vorrichtung zum Desinfizieren von Textilien, wobei die Vorrichtung mit Textilien und ggf. einer wässerigen Lösung bzw. Waschwasser in Kontakt gebracht wird, und die Vorrichtung über die wenigstens eine, vorzugsweise wenigstens zwei, UV-Licht emittierende(n) Strahlungsquelle(n) ultraviolettes Licht emittiert, so dass die Textilien und ggf. die wässerige Lösung von dem emittierten ultravioletten Licht zumindest teilweise durchdrungen werden.

Besonders bewährt hat sich dabei eine Verwendung, bei der die Vorrichtung zusammen mit Textilien in eine gebräuchliche Waschmaschine verbracht wird und mittels der Waschmaschine ein üblicher Waschvorgang durchgeführt wird.

Unter UV-Licht im Sinne der Erfindung wird insbesondere Licht der Wellenlänge 1-380 nm verstanden. Unter UV-A-Licht wird insbesondere Licht der Wellenlänge 315-380 nm verstanden. Unter UV-B-Licht wird insbesondere Licht der Wellenlänge 280-315 nm verstanden. Unter UV-C-Licht wird insbesondere Licht der Wellenlänge 100-280 nm verstanden, wobei UV-C-Licht der Wellenlänge 200-280 nm besonders geeignet ist. Aber auch UV-C-Licht der Wellenlänge der 100-200 nm ist für verschiedene Anwendungen geeignet. Für die unterschiedlichsten Anwendungen ist auch UV-A Licht oder UV-B-Licht geeignet.

Besonders bevorzugt wird das nicht erfindungsgemäße Verfahren durchgeführt, indem die Textilien während eines üblichen Waschvorgangs mit dem UV-Licht bestrahlt werden, wobei der Waschvorgang vorzugsweise mittels einer gebräuchlichen Waschmaschine durchgeführt wird.

Die Wirkung von UV-Licht bestimmter Wellenlängen ist, bezogen auf ihre desinfizierende Wirkung, wissenschaftlich belegt. Der mittels der Erfindung ermöglichte Einsatz im Reinigungsbereich ist der Sicherung der Naturressourcen, insbesondere der Wasserreserven, geschuldet und ein Schritt in Richtung der Verbesserung der Umweltsituation. Dies wird insbesondere durch die mittels der Erfindung mögliche Reduzierung von Desinfektionsmitteln, Waschmittel und Weichspüler und die damit einhergehende Reduzierung von Schadstoffeintrag in das (Ab-) Wasser erreicht.

Das erfindungsgemäße Desinfektionssystem verhindert, dass Mikroorganismen, welche sich in der Wäsche und/oder in wäscheähnlichen Gebrauchsgegenständen befinden, weiterhin infizierend wirksam sein können.
Der Belastung unserer Abwässer mit Chemikalien, wird mit Hilfe der Erfindung entgegen gewirkt. Ein auf der Erfindung basierendes Produkt beinhaltet z.B. neben einer Bedienungsanleitung einen Energiespeicher (Akku), eine Ladestation und ein Gehäuse, in dem die UV-Licht emittierenden Strahlungsquellen (UV-LEDs) untergebracht sind sowie eine Möglichkeit das Produkt in Betrieb zu nehmen sowie es außer Betrieb zu nehmen (bspw. ein Schalter oder eine Steuer- und/oder Regelungselektronik zum Ein- bzw. Ausschalten). Mögliche Einsatzorte dieses Produkts sind unter anderem Krankenhäuser, Großwäschereien, Waschsalons, Privathaushalte usw.

Weitere vorteilhafte Merkmale und Eigenschaften werden auch aus den nachfolgend gegebenen Ausführungsbeispielen ersichtlich.

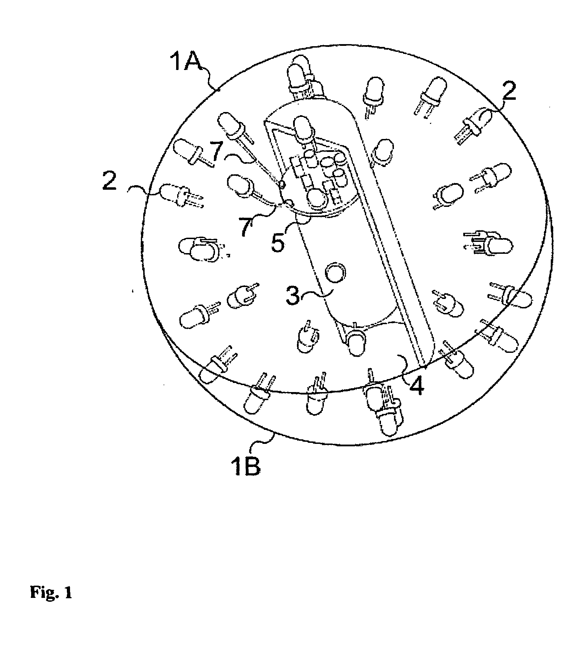

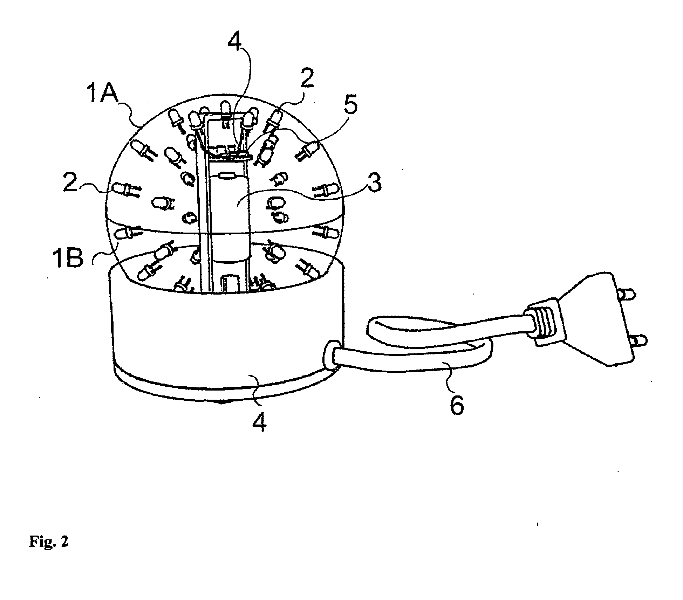

In einer geeigneten Umsetzung betrifft die Erfindung eine UV-Waschvorrichtung zum Desinfizieren von Wäsche und wäscheähnlichen Gebrauchsgegenständen, die als UV-Desinfektionswaschkugel hergestellt ist (Fig. 1). Sie besteht u.a. aus einem wasserdichten Gehäuse (Pos.1A und 1B). In dem Gehäuse befindet sich eine elektronische Steuereinheit (Pos.5), ein Akku oder ggf. mehrere Akkus (Pos.3). Dieser wenigstens eine Akku wird durch ein Energiesystem, welches sich in der Ladeschale (Pos.4) befindet, im Induktionsverfahren wieder aufgeladen (Fig. 2). Des weiteren aus einer Ladestation mit Netzanschluss und einem System zum Ein- und Ausschalten.

Im Gehäuse der Erfindung befinden sich eine oder mehrere UV-Licht emittierenden Strahlungsquellen (UV-LEDs) in bestimmten Abständen zueinander, welche durch Verbindungskabel mit der Steuerelektronik verbunden sind (Pos.5).

Mittels einer gebräuchlichen Wachmaschine wurden mehrere vergleichbare Wäschvorgänge mit gebrauchten Textilien durchgeführt, wobei der Waschvorgang jeweils mit oder ohne Beisein der UV-Lichtquelle emittierenden UV-Waschvorrichtung in der Wäschetrommel der Waschmaschine durchgeführt wurde.
Anschließend wurde die Reinheit der Textilien bzgl. der Mikrobenlast mikrobiologisch untersucht. Die Textilien, die zusammen mit der UV-Waschvorrichtung gewaschen worden waren, zeigten eine deutlich geringere Mikrobenlast als die Textilen, die ohne die UV-Waschvorichtung gewaschen worden waren.

Ebenso konnte die vorteilhafte Wirkung einer in der Waschmaschine integrierten UV-Licht emittierende Strahlungsquelle in der Praxis gezeigt werden.

Legende zu den Abbildungen (Fig. 1 und Fig. 2):
1 A - Gehäuse Teil 1 Oberschale
1B - Gehäuse Teil 2 Unterschale
2 - UV- Strahlungsquelle (UV-LED)
3 - Akkumulator
4- Ladevorrichtung - Aufnahmegehäuse: Elektronik, Verbindungskabel, Induktionsladevorrichtung
5 - Steuerelektronik
6 - Elektrokabel 220V
7 - Verbindungskabel der Strahlungsquellen (Aufgrund der Übersichtlichkeit lediglich zwei Verbindungskabel dargestellt)

## Patentansprüche

1. Vorrichtung zum Desinfizieren von Textilien, bestehend aus oder mit einem Gehäuse (1), das wenigstens eine, vorzugsweise wenigstens zwei UV- Licht emittierende Strahlungsquellen (2), elektrisch leitende Verbindungsmittel (7) und einen Akkumulator (3) trägt oder umfasst, wobei die wenigstens eine, vorzugsweise wenigstens zwei UV-Licht emittierenden Strahlungsquellen (2) im Bereich der Oberfläche des Gehäuses (1) angeordnet sind und über die elektrisch leitenden Verbindungsmittel (7) mit dem Akkumulator (3) verbunden sind, **dadurch gekennzeichnet, dass**
- das Gehäuse (1) als Ellipsoid aus gebildet ist.

2. Vorrichtung nach Anspruch 1, **dadurch gekennzeichnet, dass** die wenigstens eine, vorzugsweise wenigstens zwei UV-Licht emittierenden Strahlungsquellen (2) als UV-Licht emittierende Leuchtkörper oder vorzugsweise als UV- Licht emittierende Dioden gebildet sind.

3. Vorrichtung nach einem der vorangehenden Ansprüche, **dadurch gekennzeichnet, dass** die elektrisch leitende Verbindungsmittel (7) als Drähte oder vorzugsweise als Kabel gebildet sind, insbesondere aus einem Metall, vorzugsweise Kupfer oder Aluminium oder Legierungen eines Metalls.

4. Vorrichtung nach einem der vorangehenden Ansprüche, **dadurch gekennzeichnet, dass** das Gehäuse (1) ferner eine zumindest teilweise elektrisch leitende Halterung für den Akkumulator (3), vorzugsweise ein Akkuaufnahmegehäuse, umfasst und der Akkumulator (3) über die Halterung und die Verbindungsmittel (7) elektrisch leitend mit wenigstens zwei UV-Licht emittierenden Strahlungsquellen (2) verbunden ist.

5. Vorrichtung nach einem der vorangehenden Ansprüche, **dadurch gekennzeichnet, dass** das Gehäuse (1) als dreidimensionaler Körper gebildet ist, der Aussparungen zur Aufnahme der UV-Licht emittierenden Strahlungsquelle(n) (2), der elektrisch leitenden Verbindungmittel (7) und des Akkumulators (3) und ggf. der Halterung aufweist.

6. Vorrichtung nach einem der vorangehenden Ansprüche, **dadurch gekennzeichnet, dass** das Gehäuse aus mehreren Teilen, vorzugsweise aus einer Oberschale und einer Unterschale, zusammengesetzt ist.

7. Vorrichtung nach einem der vorangehenden Ansprüche, **dadurch gekennzeichnet, dass** die UV-Licht emittierende(n) Strahlungsquelle(n) (2) und/oder der Akkumulator (3) und ggf. die Halterung im Wesentlichen von dem Gehäuse umgeben ist/sind, wobei wenigstens ein Teil des Akkumulators (3) aus dem Gehäuse (1) und ggf. der Halterung herausragt oder nicht mit dem Gehäuse (1) in Berührung ist, so dass ein Anschluss des Akkumulators (3) an eine Ladevorrichtung (4), insbesondere an eine externe Ladevorrichtung (4) ermöglicht ist.

8. Vorrichtung nach einem der vorangehenden Ansprüche, **dadurch gekennzeichnet, dass** das Gehäuse (1) im Bereich des für den Anschluss an eine Ladevorrichtung vorgesehenen Teils des Akkumulators (3) flach/abgeschrägt ausgebildet ist.

9. Vorrichtung nach einem der vorangehenden Ansprüche, **dadurch gekennzeichnet, dass** das Gehäuse (1) aus Kunststoff, vorzugsweise durchsichtigem Kunststoff, insbesondere aus PET oder Plexiglas, gebildet ist.

10. Vorrichtung nach einem der vorangehenden Ansprüche, ferner umfassend eine Steuerelektronik zum Steuern und/oder Regeln der UV-Licht emittierenden Strahlungsquellen (2).

11. Vorrichtung nach einem der vorangehenden Ansprüche, ferner umfassend eine Ladevorrichtung (4), die vorzugsweise eine Induktionsladevorrichtung ist.

12. Vorrichtung nach einem der vorangehenden Ansprüche, **dadurch gekennzeichnet, dass** das Gehäuse (1) als Kugel gebildet ist.

13. Vorrichtung nach einem der vorangehenden Ansprüche, ferner umfassend eine Ladevorrichtung zum Laden des Akkumulators (3), enthaltend einen Rotor der auf einer Generatorwelle befestigt ist und bei Bewegung der Vorrichtung den Exzenter auf der Welle in Rotation versetzt, wobei im Generator eine Ladespannung erzeugt wird.

14. Verwendung einer Vorrichtung nach einem der vorangehenden Ansprüche
zum Desinfizieren von Textilien, wobei die Vorrichtung mit Textilien und ggf. einer wässerigen Lösung in Kontakt gebracht wird, und die Vorrichtung über die wenigstens eine, vorzugsweise wenigstens zwei, UV-Licht emittierenden Strahlungsquellen ultraviolettes Licht emittiert, so dass die Textilien und ggf. die wässerige Lösung von dem emittierten ultravioletten Licht zumindest teilweise durchdrungen werden, **dadurch gekennzeichnet, dass** die Vorrichtung zusammen mit Textilien in eine Waschmaschine verbracht wird und mittels der Waschmaschine ein üblicher Waschvorgang durchgeführt wird.

## Claims

1. Device for the disinfection of textiles, consisting of or comprising a housing (1) which carries or comprises at least one, preferably at least two UV light-emitting radiation sources (2), electrically conductive connecting means (7) and an accumulator (3), wherein said at least one, preferably at least two UV light-emitting radiation sources (2) being arranged in the region of the surface of the housing (1) and connected to the accumulator (3) by the electrically conductive connecting means (7),
**characterized in that** the housing is formed (1) as an ellipsoid.

2. Device according to claim 1, **characterized in that** the at least one, preferably at least two UV light-emitting radiation sources (2) are formed as UV light-emitting lighting bodies or, preferably as UV light-emitting diodes.

3. Device according to any one of the preceding claims, **characterized in that** the electrically conductive connecting means (7) are formed as wires or, preferably as cable, in particular of metal, preferably copper or aluminum or alloys of a metal.

4. Device according to any one of the preceding claims, **characterized in that** the housing (1) further comprises an at least partially electrically conducting support for the accumulator (3), preferably an accumulator-carrying housing, and wherein the accumulator (3) is connected via the support and the connecting means (7) to at least two UV light-emitting radiation sources (2).

5. Device according to any one of the preceding claims, **characterized in that** the housing (1) is formed as a three-dimensional body, comprising recesses for receiving the UV light-emitting radiation source(s) (2), the electrically conductive connecting means (7) and the accumulator (3) and where applicable the support.

6. Device according to any one of the preceding claims, **characterized in that** the housing is assembled of several parts, preferably of an upper shell and a lower shell.

7. Device according to any one of the preceding claims, **characterized in that** the UV light-emitting radiation source(s) (2) and / or the accumulator (3) and where applicable the support is / are substantially surrounded by the housing, where at least a portion of the accumulator (3) protrudes from the housing (1) and where applicable from the support, or is not in contact to the housing (1), in a way that a connection of the accumulator (3) to a charging device (4), in particular to an external charging device (4) is permitted.

8. Device according to any one of the preceding claims, **characterized in that** the housing (1) is shaped flat / beveled in the region of the accumulator (3) specified for the connection to a charging device.

9. Device according to any one of the preceding claims, **characterized in that** the housing (1) is composed of plastic, preferably transparent plastic material, particularly of PET or Plexiglas (PMMA).

10. Device according to any one of the preceding claims, further comprising open and / or closed loop control electronics for controlling and / or regulating the UV emitting radiation sources (2).

11. Device according to any one of the preceding claims, further comprising a charging device (4), which is preferably an induction charging apparatus.

12. Device according to any one of the preceding claims, **characterized in that** the housing (1) is formed as a sphere.

13. Device according to any one of the preceding claims, further comprising a charging device for charging the accumulator (3), comprising a rotor which is mounted on a generator shaft and which effects rotation of an eccentric on the shaft during movement of the device, whereat a charging voltage is generated in the generator.

14. Use of a device according to any one of the preceding claims for disinfection of textiles, at which said apparatus is brought into contact with textiles and where applicable with aqueous solution, and the device emits ultraviolet light via the at least one, preferably at least two, UV-light-emitting radiation source(s), in a form that the textiles and where applicable the aqueous solution is at least partially penetrated by the emitted ultraviolet light,
**characterized in that** the device along with textiles is supplied to a washing machine and a conventional washing process is carried out using the washing machine.

## Revendications

1. Dispositif pour la désinfection de textiles, composé d'un boîtier (1), qui contient ou comprend au moins une, de préférence deux sources de rayonnement ultraviolet (2), des moyens de liaison électro-conducteurs (7) et un accumulateur (3), étant entendu qu'au moins une, de préférence au moins deux sources de rayonnement ultraviolet (2) sont situées à la surface du boîtier (1) et sont reliées via les moyens de liaison électro-conducteurs (7) avec l'accumulateur (3), **caractérisé par**
- **le fait que** le boîtier (1) est conçu comme un ellipsoïde.

2. Dispositif selon la revendication 1, **caractérisé en ce qu'**au moins une, de préférence au moins deux sources de rayonnement ultraviolet (2) sont formées comme luminaires émettant des UV ou de préférence des diodes émettant des UV.

3. Dispositif selon l'une des revendications ci-dessus, **caractérisé en ce que** les moyens de liaison électro-conducteurs (7) sont formés comme des fils ou, encore avantageusement comme câbles, en particulier en métal, de préférence en cuivre ou en aluminium ou à base d'alliages d'un métal.

4. Dispositif selon l'une des revendications ci-dessus, **caractérisé en ce que** le boîtier (1) contient en plus au moins un support au moins partiellement électro-conducteur pour l'accumulateur (3), de préférence un boîtier de logement pour l'accu et que l'accumulateur (3) est relié via le support et les moyens de liaison (7) électro-conducteurs à deux sources de rayonnement ultraviolet (2) au minimum.

5. Dispositif selon l'une des revendications ci-dessus, **caractérisé en ce que** le boîtier (1) est conçu comme un corps tridimensionnel présentant des évidements pour accueillir la(les) source(s) de rayonnement ultraviolet (2), les moyens de liaison électro-conducteurs (7), l'accumulateur (3) et, le cas échéant, le support.

6. Dispositif selon l'une des revendications ci-dessus, **caractérisé en ce que** le boîtier est composé de plusieurs éléments, de préférence d'une coque supérieure et d'une coque inférieure.

7. Dispositif selon l'une des revendications ci-dessus, **caractérisé en ce que** la (les) source(s) de rayonnement ultraviolet (2) et/ou l'accumulateur (3) et, le cas échéant, le support sont essentiellement entourés par le boîtier (1), étant entendu qu'une partie de l'accumulateur au moins dépasse du boîtier (1) et, le cas échéant du support, ou n'est pas en contact avec le boîtier (1), de telle manière qu'un raccord de l'accumulateur (3) avec un dispositif de chargement (4), notamment un chargeur externe, soit possible.

8. Dispositif selon l'une des revendications ci-dessus, **caractérisé en ce que** le boîtier (1) présente un méplat au niveau de la partie de l'accumulateur (3) prévue pour le raccord avec la station de chargement.

9. Dispositif selon l'une des revendications ci-dessus, **caractérisé en ce que** le boîtier (1) est fabriqué en plastique, de préférence en plastique transparent, notamment en PET ou plexiglas.

10. Dispositif selon l'une des revendications ci-dessus, comprenant en outre une commande électronique pour contrôler et/ou régler les sources de rayonnement ultraviolet (2).

11. Dispositif selon l'une des revendications ci-dessus, comprenant en outre une station de chargement (4) qui est de préférence un dispositif de chargement à induction.

12. Dispositif selon l'une des revendications ci-dessus, **caractérisé en ce que** le boîtier (1) est conçu en forme de boule.

13. Dispositif selon l'une des revendications ci-dessus, comprenant en outre un dispositif de chargement pour recharger l'accumulateur (3), contenant un rotor fixé sur un arbre de générateur et entraînant l'excentrique sur l'arbre en rotation lorsque le dispositif est en mouvement, étant entendu qu'une tension de charge est générée dans le générateur.

14. Utilisation du dispositif selon l'une des revendications ci-dessus
pour la désinfection de textiles, étant entendu que le dispositif est mis en contact avec des textiles et, le cas échéant, une solution aqueuse, et que le dispositif émet une lumière ultraviolette à partir au minimum d'une, de préférence deux, source(s) de rayonnement ultraviolet, de telle manière que les textiles et, le cas échéant, la solution aqueuse, soient traversés au moins partiellement par la lumière ultraviolette émise, **caractérisé en ce que** le dispositif est placé dans une machine à laver usuelle avec les textiles et qu'un cycle de lavage normal est réalisé à l'aide de la machine à laver.
